# EUROPEAN PATENT APPLICATION

(11) **EP 3 901 149 A1**
(43) Date of publication of application: **27.10.2021**
(21) Application number: 19900731.1
(22) Date of filing: 16.12.2019
(51) Int. Cl.: C07D 413/14, C07D 401/12, C07D 403/12, C07D 221/04, C07D 217/00

(54) **IMPURITIES OF AMIDE DERIVATIVES AND USE THEREOF**

(30) Priority: 17.12.2018 CN 201811545845
(71) Applicant: NHWA Pharma Corporation, Xuzhou, Jiangsu 221000 (CN)
(72) Inventor: DOU, Fei, Xuzhou, Jiangsu 221152 (CN); JING, Peng, Xuzhou, Jiangsu 221152 (CN)
(74) Representative: Müller, Christian Stefan Gerd
(86) International application number: PCT/CN2019/125643
(87) International publication number: WO 2020/125580

(57) **Abstract**

An impurity A, an impurity B and an impurity G and preparation methods therefor, and an application as a reference standard for quality control of a compound as represented by formula VI.

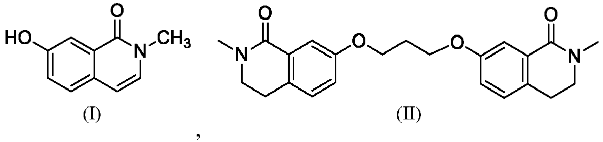

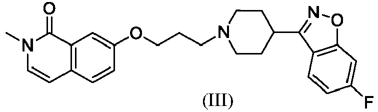

## Description

### TECHNICAL FIELD

The present invention relates to impurities of an amide-like derivatives and use thereof in the pharmaceutical industry.

### BACKGROUND

Schizophrenia is the most serious and harmful disease among all mental diseases, with a global incidence of about 1-2%. The lifetime prevalence of schizophrenia is 0.7-0.8%, which is not significantly related to gender, race, or social boundaries, and the mortality rate of schizophrenia patients is 2 to 3 times higher than that of the general population. The latest research shows that mental disease ranks first among various diseases in China in terms of the social burden caused, surpassing cardiovascular and cerebrovascular diseases, respiratory diseases, malignant tumors, and other diseases.

The patent WO2017084627A discloses a compound that acts on dopamine D2, 5-HT_{1A} and 5-HT2A receptors and a preparation method therefor. The compound has good anti-neurological disease activity and its structure is as follows:

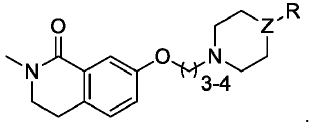

In order to ensure the safety of medication, each impurity in the active pharmaceutical ingredient must be assessed for safety, that is, impurity limits for safety must be established. According to the requirements of the International Conference on Harmonization of Technical Standards for Registration of Pharmaceuticals for Human Use (ICH), if the amount of a single impurity in an API exceeds 0.05%, the impurity should be reported; if the amount of a single impurity exceeds 0.1%, the impurity should be identified; and if the amount of a single impurity exceeds 0.15%, data to prove the safety of the impurity should be provided.

In the preparation of the compound of formula VI, it was found that the compounds of formula VI prepared by different raw materials and different production processes have different impurities. Therefore, it is necessary to confirm the structure and control the content of the new impurities, so as to meet the requirements for drug preparation, lay a foundation for toxicological research and also provide reference for the control of process synthesis conditions.

### SUMMARY

The present invention relates to an impurity A, an impurity B and an impurity G of the compound of formula VI, the preparation methods therefor, and the application as a reference standards for the quality control of the compound of formula VI,

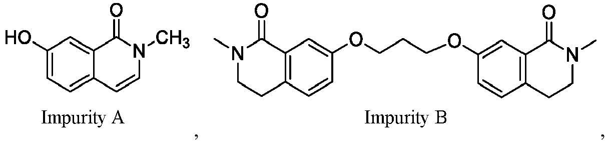

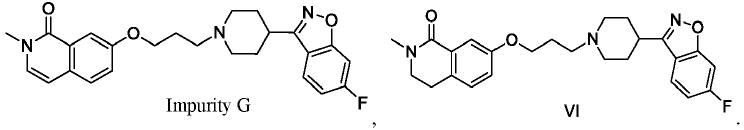

The present invention provides an impurity B of a compound of formula VI, wherein the impurity B has a structure as follows:

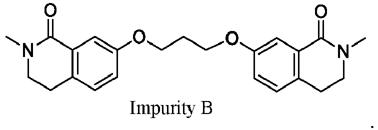

The present invention further provides a method for preparing the impurity B of the compound of formula VI, which comprises the following steps: 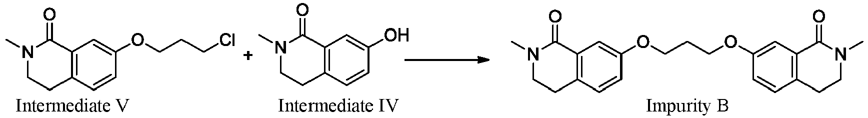
1) dissolving intermediate IV in a protic solvent;
2) dissolving a strong base in a protic solvent;
3) allowing the solution obtained in step 2) to react with the solution of step 1) to give a strong base salt of intermediate IV; and
4) allowing the solid obtained in step 3) to react with intermediate V in an inert organic solvent to give the impurity B.

In an embodiment of the present invention, the protic solvent is selected from one or more of C₁₋₄ alcohols and water. In an embodiment of the present invention, the C₁₋₄ alcohols are selected from methanol, ethanol, 1-propanol, isopropanol, *n*-butanol and 2-butanol, and preferably methanol and ethanol. In an embodiment of the present invention, the C₁₋₄ alcohols are preferably anhydrous.

In an embodiment of the present invention, the strong base is selected from NaOH, KOH, LiOH, NaH, KH and LiH.

In an embodiment of the present invention, in order to ensure the yield and the complete reaction in step 3), the reaction time may be 8-12 h.

In an embodiment of the present invention, the inert solvent is selected from one or more of ketones, ethers, halogenated hydrocarbons, nitriles, and C₅₋₁₀ saturated hydrocarbons. In an embodiment of the present invention, the inert solvent is further selected from one or more of *N*-methyl-2-pyrrolidone (NMP), *N*,*N*-dimethylformamide (DMF) and dimethyl sulfoxide (DMSO).

In an embodiment of the present invention, the ketones are selected from acetone, 2-butanone, pentan-2-one, pentan-3-one and hexan-2-one and hexan-3-one; the ethers are selected from methyl *tert*-butyl ether, diethyl ether, tetrahydrofuran, diisopropyl ether and 1,4-dioxane; the nitriles are selected from acetonitrile; the halogenated hydrocarbons are selected from dichloromethane and chloroform; the C₅₋₁₀ saturated hydrocarbons are selected from *n*-pentane, *n*-hexane, cyclohexane and *n*-heptane.

In an embodiment of the present invention, in order to ensure the yield and complete the reaction in step 4), the reaction time may be 4-8 h.

The present invention further provides a method for preparing the impurity B of the compound of formula VI, which comprises the following steps:

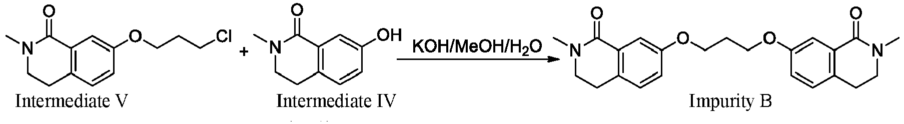

The present invention provides an impurity A of a compound of formula VI, wherein the impurity A and the compound of formula VI have structures as follows:

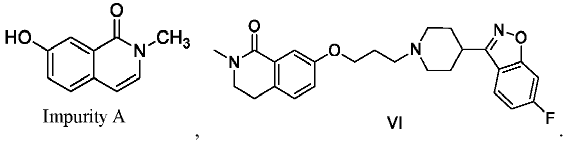

The present invention provides a method for preparing the impurity A of the compound of formula VI, which comprises the following steps: 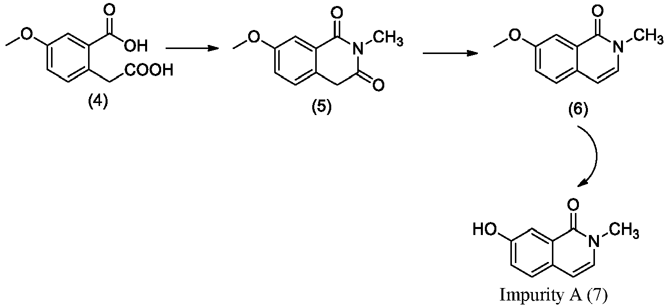 wherein,
a compound of formula (4) is dissolved in a protic solvent, and then methylamine is introduced to give a compound of formula (5);
the compound of formula (5) is dissolved in a protic solvent, and in the presence of a reducing agent, a compound of formula (6) is prepared; and
the compound of formula (6) is reacted with hydrohalic acid to give the impurity A.

In an embodiment of the present invention, the reducing agent is selected from borohydride and hydride composite.

In an embodiment of the present invention, the hydrohalic acid is selected from HF, HCl, HBr and HI; the borohydride is selected from sodium borohydride, potassium borohydride and lithium borohydride, and preferably sodium borohydride; the hydride composite is selected from lithium aluminum hydride, sodium aluminum hydride and potassium aluminum hydride; the protic solvent is selected from C₁₋₄ alcohols.

In an embodiment of the present invention, the C₁₋₄ alcohols are selected from methanol, ethanol, 1-propanol, isopropanol, *n*-butanol and 2-butanol, and preferably methanol and ethanol.

In an embodiment of the present invention, the hydrohalic acid is generally an aqueous hydrohalic acid solution.

In an embodiment of the present invention, the process of dissolving the compound of formula (5) in a protic solvent and preparing the compound of formula (6) in the presence of a reducing agent may further comprise a catalyst, wherein the catalyst is selected from dilute hydrochloric acid.

In an embodiment of the present invention, in order to make the reaction complete and achieve a good yield, the molar ratio of the compound of formula (5) to the borohydride may be 1:8-1:12, and preferably 1:3.

In an embodiment of the present invention, in order to make the reaction complete and achieve a good yield, the reaction time may be 1-6 days, and preferably 3-5 days.

The present invention further provides a method for preparing the compound of formula (4), which comprises the following steps: 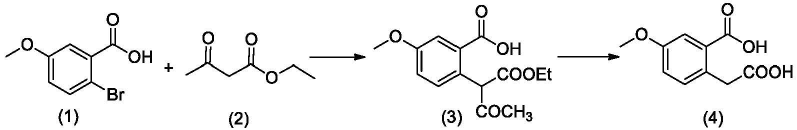 wherein,
a compound of formula (1) is reacted with a compound of formula (2) in the presence of a catalyst and a strong base to give a compound of formula (3); and
the compound of formula (3) is reacted in the presence of a strong base to give the compound of formula (4).

In an embodiment of the present invention, the catalyst is selected from metal salts, preferably CuBr, CuCl, CuI, FeCl₂, FeSO4 and FeBr2, and more preferably CuBr.

In an embodiment of the present invention, the strong base is selected from NaOH, KOH, LiOH, NaH, KH and LiH.

According to one aspect of the present invention, provided is a method for preparing the impurity A of the compound of formula VI, which comprises the following steps:

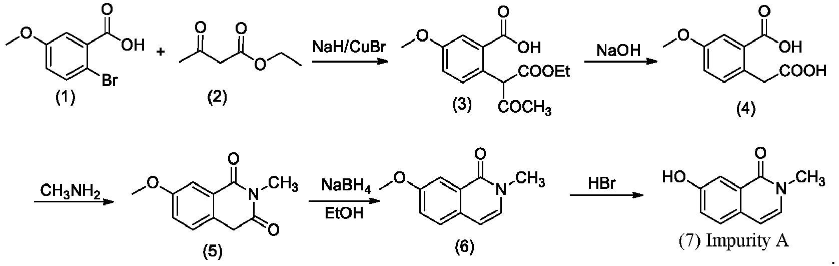

The present invention provides an impurity G of a compound of formula VI, wherein the impurity G has a structure as follows:

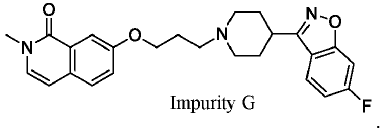

The present invention further provides a method for preparing the impurity G of the compound of formula VI, which comprises the following steps: 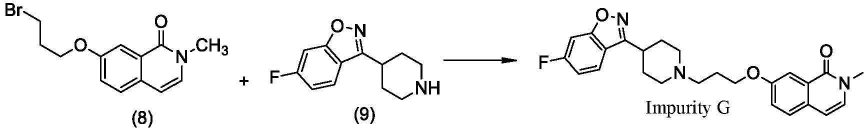 wherein the compound of formula (8) and the compound of formula (9) are reacted in the presence of a carbonate, an iodide and an organic solvent to give the impurity G.

In an embodiment of the present invention, the carbonate is selected from K₂CO₃, Na₂CO₃, Li₂CO₃ and Cs₂CO₃; the iodide is selected from KI, NaI and LiI.

In an embodiment of the present invention, the organic solvent is selected from nitriles and ketones, wherein the nitriles are selected from acetonitrile, and the ketones are selected from acetone, 2-butanone, pentan-2-one, pentan-3-one, hexan-2-one and hexan-3-one.

In an embodiment of the present invention, the compound of formula (9) also includes salts formed by the compound of formula (9) with an acid, such as hydrochloride and sulfate.

The present invention further provides a method for preparing the compound of formula (8), which comprises the following steps: 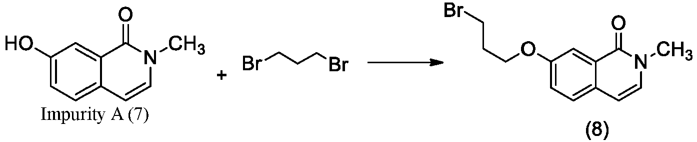 wherein the impurity A and 1,3-dibromopropane are reacted in the presence of a carbonate, an onium salt phase transfer catalyst and an organic solvent to give the compound of formula (8). In an embodiment of the present invention, the onium salt phase transfer catalyst is selected from tetraethylammonium chloride, tetrabutylammonium bromide (TBAB), tetrabutylammonium chloride, tetrabutylammonium iodide, benzyltriethylammonium chloride (TEBA), trioctylammonium chloride (TCMAC) and cetyltrimethylammonium bromide (CTMAB), and preferably benzyltriethylammonium chloride.

In an embodiment of the present invention, the organic solvent is selected from nitriles and ketones, wherein the nitriles are selected from acetonitrile, and the ketones are selected from acetone, 2-butanone, pentan-2-one, pentan-3-one, hexan-2-one and hexan-3-one.

In an embodiment of the present invention, in order to make the reaction complete and achieve a good yield, the molar ratio of the impurity A to 1,3-dibromopropane is selected from 1:2 to 1:4, and preferably 1:3.

The present invention further provides a method for preparing the impurity G of the compound of formula VI, which comprises the following steps:

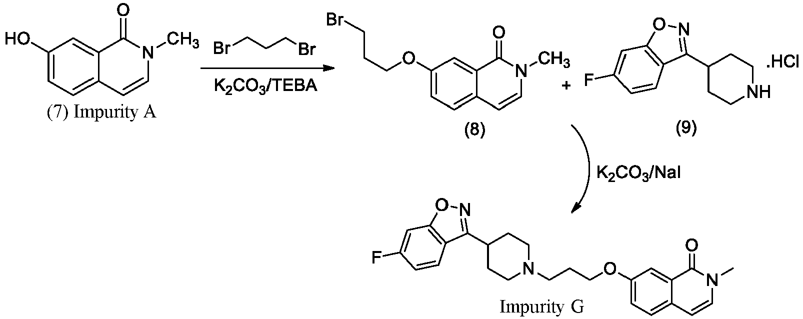

In the embodiments of the present invention, the "selected from" refers to being selected from any one or more of the exemplified items. For example, the ketones being selected from acetone, 2-butanone, pentan-2-one, pentan-3-one, hexan-2-one and hexan-3-one means that the ketones are selected from any one or more of, and preferably one of, acetone, 2-butanone, pentan-2-one, pentan-3-one, hexan-2-one and hexan-3-one.

The present invention provides use of the impurity A of the compound of formula VI in the quality control of the compound of formula VI, wherein the impurity A is used as a control substance.

The present invention further provides a method for determining the impurity A of the compound of formula VI, which comprises the following steps:
1) providing a test sample of the compound of formula VI, a self-control substance, and a marker of the impurity A; and
2) determining the presence and/or amount of the impurity A in the compound of formula VI by determining the test sample, the self-control substance and the marker by chromatography. In an embodiment of the present invention, the method for determining the impurity A of the compound of formula VI comprises:
   1) providing a test sample of the compound of formula VI;
   2) providing a marker of the impurity A;
   3) analyzing the marker of the impurity A by HPLC to determine the retention time of the impurity A; and
   4) analyzing the test sample of the compound of formula VI by HPLC to determine whether the test sample contains substances whose retention time is basically the same as that of step 3), so as to determine the presence of the impurity A in the compound of formula VI.

The present invention provides use of the impurity B of the compound of formula VI in the quality control of the compound of formula VI, wherein the impurity B is used as a control substance.

The present invention further provides a method for determining the impurity B of the compound of formula VI, which comprises the following steps:
1) providing a test sample of the compound of formula VI, a self-control substance, and a marker of the impurity B; and
2) determining the presence and/or amount of the impurity B in the compound of formula VI by determining the test sample, the self-control substance and the marker by chromatography. In an embodiment of the present invention, the method for determining the impurity B of the compound of formula VI comprises:
   1) providing a test sample of the compound of formula VI;
   2) providing a marker of the impurity B;
   3) analyzing the marker of the impurity B by HPLC to determine the retention time of the impurity B; and
   4) analyzing the test sample of the compound of formula VI by HPLC to determine whether the test sample contains substances whose retention time is basically the same as that of step 3), so as to determine the presence of the impurity B in the compound of formula VI.

The present invention provides use of the impurity G of the compound of formula VI in the quality control of the compound of formula VI, wherein the impurity G is used as a control substance.

The present invention further provides a method for determining the impurity G of the compound of formula VI, which comprises the following steps:
1) providing a test sample of the compound of formula VI, a self-control substance, and a marker of the impurity G; and
2) determining the presence and/or amount of the impurity G in the compound of formula VI by determining the test sample, the self-control substance and the marker by chromatography. In an embodiment of the present invention, the method for determining the impurity G of the compound of formula VI comprises:
   1) providing a test sample of the compound of formula VI;
   2) providing a marker of the impurity G;
   3) analyzing the marker of the impurity G by HPLC to determine the retention time of the impurity G; and
   4) analyzing the test sample of the compound of formula VI by HPLC to determine whether the test sample contains substances whose retention time is basically the same as that of step 3), so as to determine the presence of the impurity G in the compound of formula VI.

In an embodiment of the present invention, those skilled in the art should be aware that the retention time may vary with the environment for determination, analytical instrument and other factors. The time error for the retention time of the test sample of the compound of formula VI and the retention time of the impurity marker is ±2 min, and this time error should be taken into account when confirming the retention time.

The present invention provides a compound of formula VI, wherein in the compound of formula VI, the content of the impurity A is selected from 0.01-1 wt.%. In an embodiment of the present invention, the content of the impurity A is selected from 0.01-0.5 wt.%.

The present invention provides a compound of formula VI, wherein in the compound of formula VI, the content of the impurity B is selected from 0.01-1 wt.%. In an embodiment of the present invention, the content of the impurity B is selected from 0.01-0.5 wt.%.

The present invention provides a compound of formula VI, wherein in the compound of formula VI, the content of the impurity G is selected from 0.01-1 wt.%. In an embodiment of the present invention, the content of the impurity G is selected from 0.01-0.5 wt.%.

The present invention provides use of the compound of formula VI in the preparation of a medicament for treating neuropsychiatric diseases, wherein the compound of formula VI contains the impurity A in an amount selected from 0.01-1 wt.%.

The present invention provides use of the compound of formula VI in the preparation of a medicament for treating neuropsychiatric diseases, wherein the compound of formula VI contains the impurity B in an amount selected from 0.01-1 wt.%.

The present invention provides use of the compound of formula VI in the preparation of a medicament for treating neuropsychiatric diseases, wherein the compound of formula VI contains the impurity G in an amount selected from 0.01-1 wt.%.

The present invention provides a compound of formula VI used as a medicament for neuropsychiatric diseases, wherein the compound of formula VI contains the impurity A in an amount selected from 0.01-1 wt.%.

The present invention provides a compound of formula VI used as a medicament for neuropsychiatric diseases, wherein the compound of formula VI contains the impurity B in an amount selected from 0.01-1 wt.%.

The present invention provides a compound of formula VI used as a medicament for neuropsychiatric diseases, wherein the compound of formula VI contains the impurity G in an amount selected from 0.01-1 wt.%.

In an embodiment of the present invention, the neuropsychiatric disease is schizophrenia.

### Beneficial Effects

The impurity A, impurity B and impurity G of the compound of formula VI provided herein can be used for the detection of the impurity content during the preparation of the compound of formula VI, so as to allow the API of the compound of formula VI to meet the ICH standards for human use, lay a foundation for toxicological research and also provide reference for the control of process synthesis conditions.

### BRIEF DESCRIPTION OF THE DRAWINGS

FIG. 1 is an HPLC spectrum of the marker solution of impurity A.
FIG. 2 is an HPLC spectrum of the marker solution of impurity B.
FIG. 3 is an HPLC spectrum of the marker solution of impurity G.
FIG. 4 is an HPLC spectrum of the test sample solution of the compound of formula VI.

### DETAILED DESCRIPTION

Hereinafter, the present invention will be explained in more detail in reference to examples. The examples are only used to illustrate the technical solutions of the present invention, and the essence and scope of the present invention are not limited thereto.

Test conditions for the instruments used in the experiment:
1. High performance liquid chromatograph (HPLC)
Instrument model: Agilent 1260 (DAD) binary pump liquid chromatography
Chromatographic column: SHIMADZU VP-ODS C18 column (4.6×250 mm, 5 µm)
Mobile phases:
A: 0.01 mol/L potassium dihydrogen phosphate, 0.1% triethylamine (adjust the pH to 2.5 with phosphoric acid)-methanol (90:10)
B: 0.01 mol/L potassium dihydrogen phosphate, 0.1% triethylamine (adjust the pH to 2.5 with phosphoric acid)-methanol (20:80)
Flow rate: 1.0 mL/min Column temperature: 35 °C
Wavelength: 210 nm Injection volume: 15 µL
Gradient conditions (volume ratio):

| **Time (min)** | **A (%)** | **B (%)** |
|---|---|---|
| 0 | 80 | 20 |
| 10 | 75 | 25 |
| 15 | 55 | 45 |
| 35 | 45 | 55 |
| 60 | 20 | 80 |
| 65 | 20 | 80 |
| 66 | 80 | 20 |
| 76 | 80 | 20 |

### Example 1: preparation of 7,7'-(propane-1,3-diylbis(oxy))bis(2-methyl-3,4-dihydroisoquinolin-1(2H)-one) (impurity B)

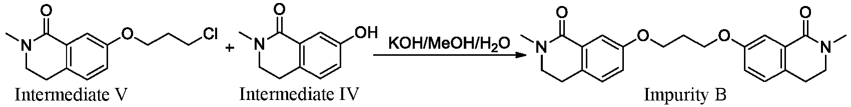

Intermediate IV (15 g, 84.65 mmol) was added to a 500 mL three-necked flask, and then methanol (300 mL) was added to dissolve it. Potassium hydroxide (6.0 g, 106.93 mmol) was weighed out and dissolved in a mixed solvent of water (50 mL) and methanol (100 mL), and the mixture was added dropwise to the reaction system. The resulting reaction system was heated to reflux and reacted for about 10 h. After the reaction completion as detected by TLC, the oil bath was removed and the reaction system was cooled down naturally. Then the solvent was removed by rotary evaporation under reduced pressure, and the residue was dried at 65 °C to give a white solid (18.4 g), which was then dissolved in DMF (300 mL) and added with intermediate V (21.5 g, 84.65 mmol). The mixture was heated to 65 °C and reacted for about 5 h. After the reaction completion as detected by TLC, the oil bath was removed, and the mixture was added with water (about 500 mL), stirred and filtered under vacuum. The aqueous phase was extracted with sufficient ethyl acetate, and the organic phase was washed with water and dried. The solvent was removed by rotary evaporation under reduced pressure, and the solids were combined, purified by column chromatography (ethyl acetate/petroleum ether = 1:1), and dried to give a white powder (28.0 g, 84.8% yield).

¹H NMR (400 MHz, CDCl₃) δ 7.33 (d, J = 6.0 Hz, 2H), 7.25 (d, J = 1.6 Hz, 2H), 7.06 (m, 2H), 4.15 (m, 4H), 3.85 (m, 4H), 3.14 (m, 4H), 3.08 (s, 6H), 2.24 (m, 2H).MS (ESI) m/z 395.2([M+H]+).

### Example 2: preparation of 2-carboxymethyl-5-methoxybenzoic acid (the compound of formula (4))

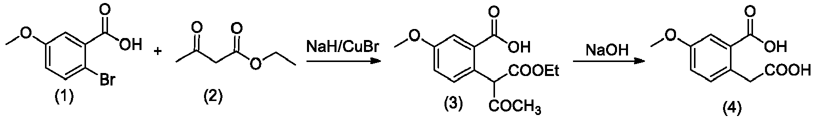

Synthesis of the compound of formula (3) (2-(1-ethoxy-1,3- dioxobutanediyl)-5-methoxybenzoic acid): Under the N2 atmosphere, 2-bromo-5-methoxybenzoic acid (50 g, 216.4 mmol), CuBr (1.9 g, 12.9 mmol, 0.06 eq) and ethyl acetoacetate (about 800 mL) were added successively, and the mixture was cooled down in an ice-salt bath. When the internal temperature dropped to below 10 °C, NaH (20.8 g, 519.4 mmol, 2.4 eq) containing mineral oil was added in portions. After the addition, the reaction system was transferred to an oil bath for heating and reacted at 80 °C for 2 h. After the disappearance of the raw materials as detected by TLC, the heating was stopped, and the reaction system was naturally cooled to room temperature. Then the reaction system was poured into water (about 1 L) and extracted with a large amount of ethyl acetate to remove ethyl acetoacetate. The resulting reaction system was then adjusted to acidic pH with HCl (1 N), extracted with ethyl acetate, washed with saturated brine and dried over anhydrous Na₂SO₄. The solvent was removed by rotary evaporation under reduced pressure to give a yellow oily liquid (69.3 g), which was directly used in the next step without purification. MS (ESI) m/z 280.1 ([M+H]+).

Synthesis of the compound of formula (4) (2-carboxymethyl-5-methoxybenzoic acid): NaOH solution (680 mL, 2 N) was added to the yellow oily liquid of the compound of formula (3), and then the reaction system was stirred at room temperature. After the reaction completion as detected by TLC about 2 h later, HCl (1 N) was added dropwise to the reaction system slowly to adjust its pH, and then a large number of solids were precipitated. When no solid was precipitated, the dropwise addition was stopped. The solids were filtered out under vacuum, and the filter cake was washed with a large amount of purified water, and dried at 80 °C to give a light yellow powder (28 g). The total yield of the above two steps was 53.9%. MS (ESI) m/z 210.1 ([M+H]⁺).

### Example 3: preparation of 7-hydroxy-2-methylisoquinolin-1(2H)-one (impurity A)

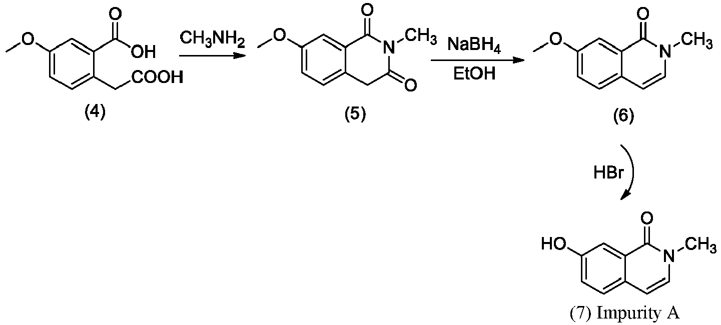

Synthesis of the compound of formula (5) (7-methoxy-2-methylisoquinolin-1,3(2*H*,4*H*)-dione):2-carboxymethyl-5-methoxybenzoic acid (10 g, 47.6 mmol) was weighed out and dissolved in anhydrous methanol (300 mL), and then dry methylamine gas was introduced for about 1 h. The reaction system, which has alkaline pH, was stirred at room temperature for 1 h. Then methanol was removed by rotary evaporation under reduced pressure, and the residue was dissolved in xylene (600 mL). The mixture was heated to reflux, a water separator was provided to remove water, and the mixture was then reacted for about 10 h. After the reaction completion as detected by TLC, the heating was stopped and the mixture was naturally cooled to room temperature. Xylene was removed by rotary evaporation under reduced pressure, and the residue was purified by column chromatography (ethyl acetate/petroleum ether = 1:1) to give a light yellow powder (8.1 g, 83.5% yield). MS (ESI) m/z 205.1 ([M+H]+).

Synthesis of the compound of formula (6) (7-methoxy-2-methylisoquinolin-1(2*H*)-one): 7-methoxy-2-methylisoquinolin-1,3(2*H*,4*H*)-dione (5.0 g, 24.4 mmol) was added into a 500 mL four-necked flask and then dissolved in absolute ethanol (500 mL), and the reaction mixture was cooled down in an ice-salt bath. When the temperature drops to below 0 °C, solid NaBH₄ (9.2 g, 243.7 mmol) was added in portions. The reaction system was then reacted at about 0 °C for about 5 days, during which a diluted hydrochloric acid solution can be added dropwise to catalyze the reaction. After the reaction completion as detected by TLC, the ice bath was removed, and the reaction system was naturally warmed to room temperature, added with a large amount of diluted hydrochloric acid to quench the reaction, extracted with ethyl acetate, washed with saturated brine and dried over anhydrous Na₂SO₄. The solvent was removed by rotary evaporation under reduced pressure, and the residue was purified by column chromatography (ethyl acetate/petroleum ether = 1:1) to give a yellow oily liquid (3.3 g, 71.7% yield). MS (ESI) m/z 189.1 ([M+H]⁺).

Synthesis of impurity A (7-hydroxy-2-methylisoquinolin-1(2*H*)-one): A yellow oily liquid of 7-methoxy-2-methylisoquinolin-1(2*H*)-one (3.5 g) was weighed out and added to 48% HBr solution (40 mL), and then the reaction system was heated to about 120 °C for about 8 h. After the reaction completion as detected by TLC, the reaction system was diluted with water (200 mL), extracted with dichloromethane, washed with saturated brine and dried over anhydrous Na₂SO₄. The solvent was removed by rotary evaporation under reduced pressure, and the residue was purified by column chromatography (ethyl acetate/petroleum ether = 5:1) to give a solid (2.4 g, 75% yield). MS (ESI) m/z 176.1 ([M+H]+).

### Example 4: preparation of 7-(3-bromopropan)-2-methylisoquinolin-1(2H)-one (the compound of formula (8))

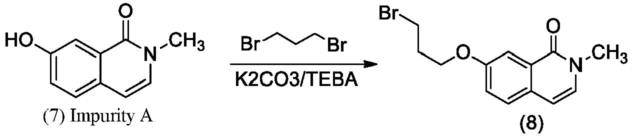

Synthesis of the compound of formula (8) (7-(3-bromopropan)-2-methylisoquinolin-1(2*H*)-one): 7-hydroxy-2-methylisoquinolin-1(2*H*)-one (2.4 g, 13.7 mmol), 1,3-dibromopropane (8.3 g, 41.1 mmol), K₂CO₃ powder (18.9 g, 137.0 mmol), benzyltriethylammonium chloride (3.1 g, 13.7 mmol) and acetone (250 mL) were added successively to a single-necked flask, and then the reaction system was heated to reflux. After the reaction completion as detected by TLC 5 h later, the heating was stopped, and the reaction system was naturally cooled to room temperature. The solid was removed by filtration under vacuum, and the filter cake was washed with acetone (about 50 mL). The liquids were combined, the solvent was removed by rotary evaporation under reduced pressure, and the residue was purified by column chromatography (ethyl acetate/petroleum ether = 1:10) to give a white solid (4 g, 99% yield). MS (ESI) m/z 295.0 ([M+H]⁺).

### Example 5: preparation of 7-(3-(4-(6-fluorobenzo[d]isoxazol-3-yl)piperidin-1-yl) propoxy)-2-methylisoquinolin-1(2H)-one (impurity G)

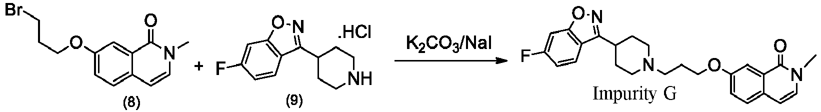

7-(3-bromopropan)-2-methylisoquinolin-1(2*H*)-one (3.85 g, 13 mmol), 6-fluoro-3-(piperidin-4-yl)benzo[*d*]isoxazole hydrochloride (5.0 g, 19.5 mmol), K₂CO₃ (5.4 g, 39 mmol), KI (0.5 g, 3.0 mmol) and acetonitrile (200 mL) were added successively to a 250 mL single-necked flask, and then the reaction system was heated to reflux. After the reaction completion as detected by TLC, the solvent was removed by filtration under vacuum, and the filter cake was washed with acetonitrile (50 mL). Then the acetonitrile was combined, and the residue was purified by column chromatography (dichloromethane/methanol = 30:1) to give a light yellow solid (2.2 g, 39.4% yield).

¹H NMR (400 MHz, CDCl₃) δ 7.86 (d, J = 2.6 Hz, 1H), 7.74 (dd, J = 8.7, 5.1 Hz, 1H), 7.45 (d, J = 8.7 Hz, 1H), 7.33-7.19 (m, 2H), 7.06 (td, J = 8.9, 2.1 Hz, 1H), 6.98 (d, J = 7.3 Hz, 1H), 6.46 (d, J = 7.3 Hz, 1H), 4.19 (t, J = 6.4 Hz, 2H), 3.62 (s, 3H), 3.10 (t, J = 10.3 Hz, 3H), 2.62 (t, J = 7.3 Hz, 2H), 2.32-1.88 (m, 8H). MS (ESI) m/z 436.2([M+H]+).

### Example 6: preparation of 7-(3-(4-(6-fluorobenzo[d]isoxazol-3-yl)piperidin-1-yl) propoxy)-2-methyl-3,4-dihydroisoquinolin-1(2H)-one (the compound of formula VI)

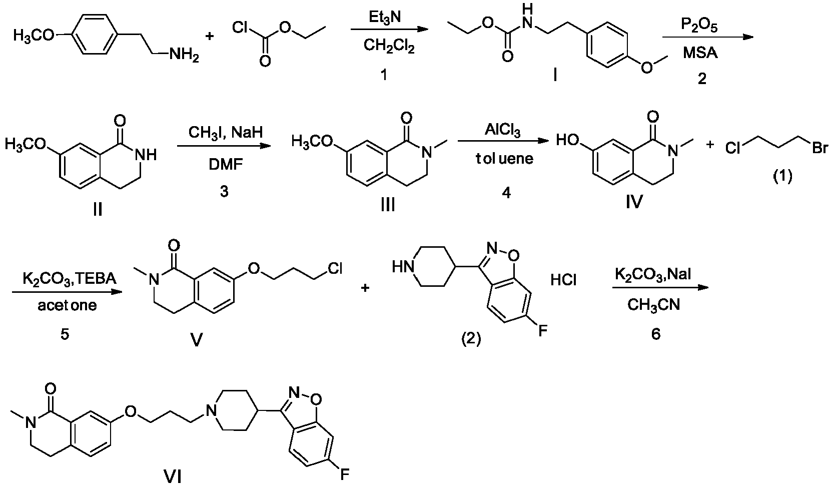

Preparation of ethyl (4-methoxyphenethyl)carbamate (intermediate I): ethyl chloroformate (275.0 g, 2.55 mol) (industrial pure, purchased from Shanghai Beihe Chemical Co., Ltd.) and dichloromethane (2500 mL) were added to a 5 L reaction flask, and then the reaction system was stirred and cooled to 0 °C in an ice-salt bath. Then the reaction system was added dropwise with a solution of 4-methoxyphenethylamine (350 g, 2.32 mol) (industrial pure, purchased from Meryer (Shanghai) Chemical Technology Co., Ltd.) in dichloromethane (1000 mL) slowly, and the temperature of the reaction system was maintained at 0-5 °C. After the dropwise addition, triethylamine (351 g, 3.48 mol) was added dropwise slowly, and the temperature of the reaction system was maintained at 5-10 °C. After the dropwise addition, the resulting reaction system was stirred at room temperature (25±5 °C) for 1 h. After the reaction was completed, the reaction system was added with water (1000 mL) to quench the reaction and then stirred for 5 min. Then the reaction system was left to stand for liquid separation to give an organic layer. The organic phase was washed successively with 1 mol/L hydrochloric acid solution (600 mL) and saturated sodium chloride solution (600 mL), dried over anhydrous sodium sulfate (150 g) for 1 h, and then filtered. The filtrate was concentrated to dryness to give a light yellow oil, which was cooled down at room temperature for 1 h to give a light yellow solid (508 g, 98.3% yield), MS (ESI) m/z 223. 3 ([M+H]+).

Preparation of 7-methoxy-3,4-dihydroisoquinolin-1(2*H*)-one (intermediate II): Phosphorus pentoxide (510.0 g, 1.80 mol) and methanesulfonic acid (2000 mL) were added to a 5 L reaction flask, and then the mixture was stirred and heated to 125-130 °C in an oil bath until phosphorus pentoxide was completely dissolved. Then the reaction system was stirred at room temperature to cool down until the temperature of the reaction system drops to 70 °C, and then added with (1000 g, 2.24 mol) intermediate I. The reaction system was heated to 125 °C in an oil bath and then reacted for 2 h. After the reaction was completed, the reaction system was cooled down and then added with ice (500 g) to quench the reaction. Then the reaction system was added with water (5000 mL) and extracted with dichloromethane (1200 mL × 6). The organic phases were combined, added with anhydrous potassium carbonate (500 g), stirred for 0.5 h, and filtered under vacuum. The solution was dried over anhydrous sodium sulfate (500 g) for 1 h and filtered under vacuum, and the filtrate was concentrated to dryness to give a brown oil, which was cooled down at 0 °C for 5 h and then filtered under vacuum. The filter cake was washed with ethyl acetate (300 mL) and dried at 50±5 °C for 8 h to give an off-white solid (436.5 g, 55.1% yield), MS (ESI) m/z 177.1 ([M+H]+).

Preparation of 7-methoxy-3,4-dihydroisoquinolin-1(2*H*)-one (intermediate III): 60% sodium hydride (54.2 g, 1.36 mol) and *N*,*N*-dimethylformamide (1000 mL) were added to a 3 L reaction flask, and then the reaction system was stirred and cooled to 0-5 °C in an ice-salt bath. Then the reaction system was added dropwise with a solution of intermediate II (200 g, 1.13 mol) in *N*,*N*-dimethylformamide (500 mL) slowly, and the temperature of the reaction system was maintained at 5-10 °C. After the dropwise addition, methyl iodide (176.5 g, 1.24 mol) was added dropwise slowly, and the temperature of the reaction system was maintained at 10-15 °C. After the dropwise addition, the reaction system was stirred at room temperature (25±5 °C) for 1 h. Then the reaction system was added dropwise with water (500 mL) to quench the reaction, mixed with water (4 L), added with sodium chloride (1630 g) to be saturated, and then extract with ethyl acetate (800 mL × 6). The organic phases were combined, washed with saturated sodium chloride solution (500 mL × 3), dried over anhydrous sodium sulfate (300 g) for 1 h, and filtered. The filtrate was concentrated to dryness to give a brown-yellow oil (258 g, over 100% yield), MS (ESI) m/z 191.1 ([M+H]+).

Preparation of 7-hydroxy-2-methyl-3,4-dihydroisoquinolin-1(2*H*)-one (intermediate IV): Anhydrous aluminum chloride (257.6 g, 1.94 mol) and toluene (1500 mL) were added to a 3 L reaction flask, and then the reaction system was stirred at room temperature (25±5 °C) and added dropwise with a solution of intermediate III (185.0 g, 0.97 mol) in toluene (300 mL) slowly. After the dropwise addition, the reaction system was heated to reflux in an oil bath (about 114-115 °C) and then reacted for 4 h under nitrogen atmosphere. After the reaction was completed, the reaction system was cooled to 70 °C at room temperature. The toluene solution was poured out, and 4 mol/L hydrochloric acid solution (2000 mL) was poured into the flask. The reaction system was added with ice to cool down, stirred at room temperature for 1 h, and then filtered. The filter cake was washed to neutral with water (500 mL), dissolved with 2 mol/L sodium hydroxide solution (1000 mL), and then washed successively with toluene (500 mL) and dichloromethane (500 mL). The aqueous layer was adjusted to pH 3-4 with 36% hydrochloric acid solution, cooled down in an ice bath, stirred for 0.5 h, and then filtered. The filter cake was washed to neutral with water (500 mL) and then dried at 50±5 °C for 12 h to give an off-white solid (162.1 g, 94.7% yield), MS (ESI) m/z 177.2 ([M+H]+).

Preparation of 7-(3-chloropropoxy)-2-methyl-3,4-dihydroisoquinolin-1(2*H*)-one (intermediate V): Intermediate IV (156.0 g, 0.88 mol), the compound of formula (1) (277.6 g, 1.76 mol), anhydrous potassium carbonate (364.9 g, 2.64 mol), benzyltriethylammonium chloride (6.0 g, 0.03 mol) and acetone (1600 mL) were added to a 3 L reaction flask, and the reaction system was heated to reflux in an oil bath (about 60-65 °C) and stirred for 6 h. After the reaction was completed, the reaction system was filtered, and the filtrate was concentrated under reduced pressure to remove the solvent, thus giving a yellow oil, which was dissolved in acetone (500 mL). The mixture was stirred at room temperature, added with *n*-hexane (3000 mL), cooled down in an ice bath, stirred for 1 h, and then filtered. The filter cake was dried at 50±5 °C for 6 h to give an off-white solid (213.2 g, 95.8% yield), MS (ESI) m/z 254.1 ([M+H]+). Preparation of 7-(3-(4-(6-fluorobenzo[*d*]isoxazol-3-yl)piperidin-1-yl)propoxy)-2-methyl-3,4-dihydroisoquinolin-1(2*H*)-one (the compound of formula VI): intermediate V (210.0 g, 0.83 mol), the hydrochloride of the compound of formula (2) (223.6 g, 0.87 mol), anhydrous potassium carbonate (344.3 g, 2.64 mol), sodium iodide (3.7 g, 0.02 mol) and acetonitrile (2100 mL) were added to a 5 L reaction flask, and then the reaction system was heated to reflux in an oil bath (about 80-85 °C) and stirred for 24 h. After the reaction was completed, the reaction system was filtered. The filtrate was stirred for 3 h in an ice bath, and filtered, and the filter cake was dried at 50±5 °C for 3 h to give a yellow solid (264.6 g, 82.6% yield), which was the crude product of the compound of formula VI. The crude product of the compound of formula VI (260.0 g), acetone (800 mL) and methanol (80 mL) were added to a 2000 mL reaction flask, and then the reaction system was heated to reflux in an water bath (about 60-65 °C), and stirred until complete dissolution. Then the reaction system was added with activated carbon (8.0 g), stirred for 10 min and filtered while being hot. The filtrate was transferred to a 2000 mL reaction flask, cooled down at room temperature (25±5 °C), stirred for 4 h, filtered under vacuum, and then washed with acetone (200 mL). The filter cake was dried at 50±5 °C for 6 h to give an off-white powder (180.3 g, 79.3% yield).

¹H-NMR (600MHz, CDCl3) δ 2.02-2.19 (m, 8H), 2.60 (t, 2H, J = 12Hz), 2.96 (t, 2H, J =12Hz), 3.09-3.11(m, 3H), 3.18(s, 3H), 3.56(t, 2H, J = 12Hz), 4.11 (t, 2H, J = 6Hz), 6.98-7.00 (m, 1H), 7.06-7.10 (m, 2H), 7.24-7.26 (m, 1H), 7.64 (d, 1H, J = 6Hz), 7.74-7.76 (m, 1H) · MS (ESI) m/z 438. 2 ([M+H]⁺).

### Example 7: HPLC determination of impurity A, impurity B and impurity G in the compound of formula VI

Preparation of solutions:
Marker solutions of impurities A, B and G: Impurities A, B and G were precisely weighed out, and each dissolved with diluent (mobile phase A: mobile phase B = 4:1) and diluted quantitatively into 0.5 mg/mL solutions, which were used as marker solutions.
Test sample solution of the compound of formula VI: The compound of formula VI (prepared according to the method described in Example 6) was precisely weighed out, dissolved with diluent (mobile phase A: mobile phase B = 4:1) and diluted quantitatively into a 1 mg/mL test sample solution of the compound of formula VI.

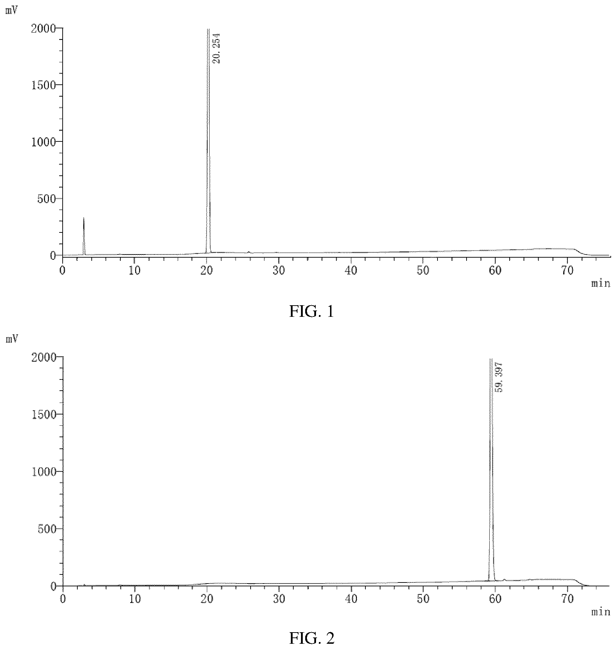

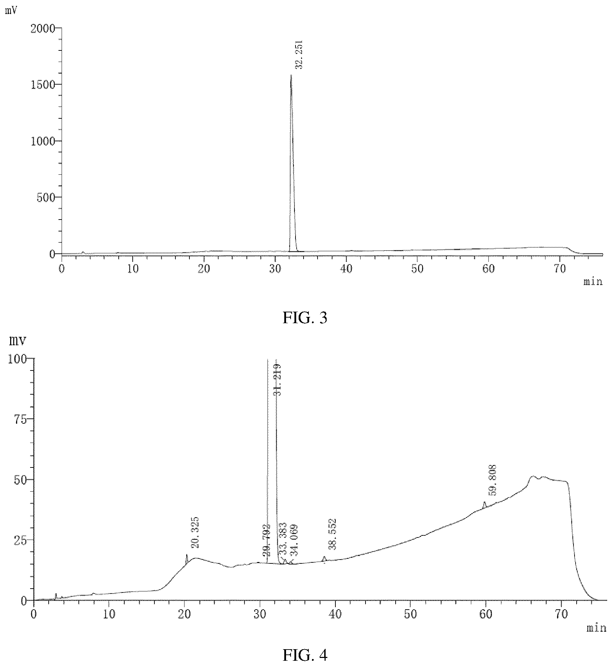

Determination method:
15 µL of each of the marker solutions of impurity A, impurity B and impurity G was precisely measured out, and injected separately into a high performance liquid chromatograph. The chromatograms were recorded to determine the peak time of each of impurity A, impurity B and impurity G. The peak time of each impurity is shown in FIGs. 1-3.
15 µL of test sample solution of the compound of formula VI was precisely measured out, and injected into a high performance liquid chromatograph. The chromatograms were recorded to determine the presence and peak area of each of impurity A, impurity B and impurity G. The presence of each impurity in the compound of formula VI is shown in FIG. 4.

Content calculation:
According to the area normalization method, the mass percentage of each impurity in the test sample of the compound of formula VI was calculated. The specific experimental results are shown in Table 1.

**Table 1. Content of each impurity in the test sample**

| **Serial number** | **Compound** | **Retention time** | **Area** | **Height** | **Area (%)** |
|---|---|---|---|---|---|
| 1 | Impurity A | 20.325 | 56652 | 5820 | 0.057 |
| 2 | / | 29.792 | 4905 | 350 | 0.005 |
| 3 | Compound of formula VI | 31.219 | 98195298 | 2934467 | 99.768 |
| 4 | Impurity G | 33.383 | 49944 | 2988 | 0.051 |
| 5 | / | 34.069 | 27736 | 1414 | 0.028 |
| 6 | / | 38.552 | 36719 | 1907 | 0.037 |
| 7 | Impurity B | 59.808 | 52551 | 3098 | 0.054 |
| Total | / | / | 98423805 | 2950044 | 100.000 |

| | | | | | |
|---|---|---|---|---|---|
| Although the present invention has been described in detail above, those skilled in the art understand that various modifications and changes can be made to the present invention without departing from the spirit and scope of the present invention. The protection scope of the present invention is not limited to the detailed description above, but should be defined by the claims. | | | | | |

## Claims

1. An impurity B of a compound of formula VI, wherein the impurity B and the compound of formula VI have structures as follows:

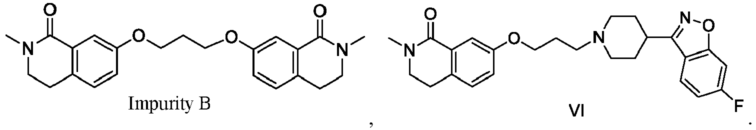

2. A method for preparing the impurity B according to claim 1, comprising the following steps: 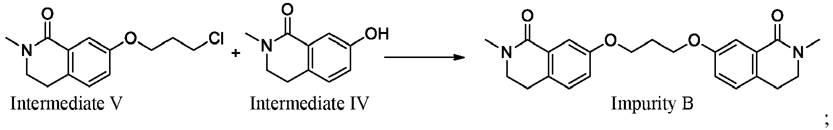
1) dissolving intermediate IV in a protic solvent;
2) dissolving a strong base in a protic solvent;
3) allowing the solution obtained in step 2) to react with the solution of step 1) to give a strong base salt of intermediate IV; and
4) allowing the solid obtained in step 3) to react with intermediate V in an inert organic solvent to give the impurity B;
wherein the protic solvent is selected from one or more of C₁₋₄ alcohols and water, and the strong base is selected from NaOH, KOH, LiOH, NaH, KH and LiH.

3. An impurity G of a compound of formula VI, wherein the impurity G and the compound of formula VI have structures as follows:

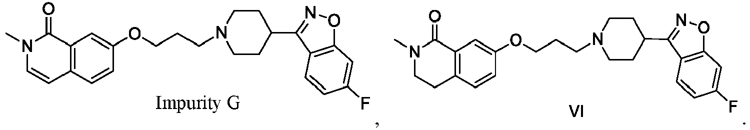

4. A method for preparing the impurity G according to claim 3, comprising the following step: 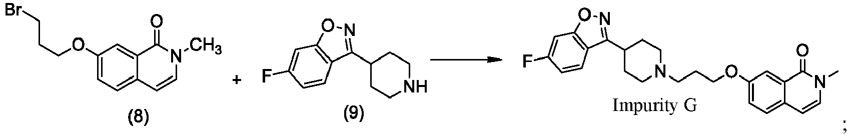 wherein the compound of formula (8) and the compound of formula (9) are reacted in the presence of a carbonate, an iodide and an organic solvent to give the impurity G; wherein
the carbonate is selected from K₂CO₃, Na₂CO₃, Li₂CO₃ and Cs₂CO₃, the iodide is selected from KI, NaI and Lil, and the organic solvent is selected from nitriles and ketones, wherein the nitriles are selected from acetonitrile, and the ketones are selected from acetone, 2-butanone, pentan-2-one, pentan-3-one, hexan-2-one and hexan-3-one.

5. The method according to claim 4, wherein the compound of formula (8) is prepared by the following step: 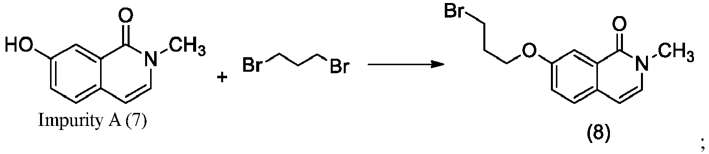 wherein the impurity A and 1,3-dibromopropane are reacted in the presence of a carbonate, an onium salt phase transfer catalyst and an organic solvent to give the compound of formula (8); wherein
the onium salt phase transfer catalyst is selected from tetraethylammonium chloride, tetrabutylammonium bromide, tetrabutylammonium chloride, tetrabutylammonium iodide, benzyltriethylammonium chloride, trioctylammonium chloride and cetyltrimethylammonium bromide, the carbonate is selected from K₂CO₃, Na₂CO₃, Li₂CO₃ and Cₛ₂CO₃, and the organic solvent is selected from nitriles and ketones, wherein the nitriles are selected from acetonitrile, and the ketones are selected from acetone, 2-butanone, pentan-2-one, pentan-3-one, hexan-2-one and hexan-3-one.

6. An impurity A of a compound of formula VI, wherein the impurity A and the compound of formula VI have structures as follows:

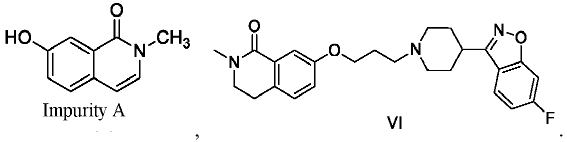

7. A method for preparing the impurity A according to claim 6, comprising the following steps: 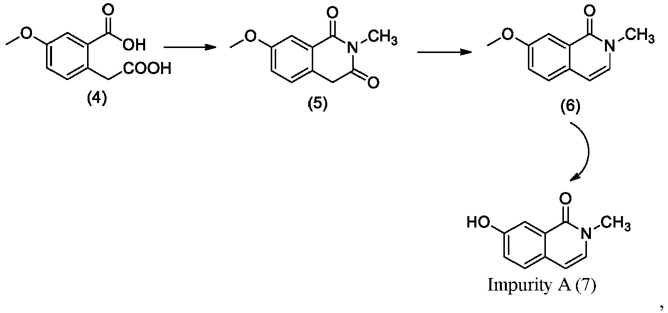 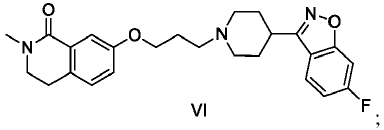 wherein,
a compound of formula (4) is dissolved in a protic solvent, and then methylamine is introduced to give a compound of formula (5);
the compound of formula (5) is dissolved in a protic solvent, and in the presence of a reducing agent, a compound of formula (6) is prepared; and
the compound of formula (6) is reacted with hydrohalic acid to give the impurity A;
wherein the reducing agent is selected from borohydride and hydride composite.

8. The method according to claim 7, wherein the hydrohalic acid is selected from HF, HCl, HBr and HI, the borohydride is selected from sodium borohydride, potassium borohydride and lithium borohydride, the hydride composite is selected from lithium aluminum hydride, sodium aluminum hydride and potassium aluminum hydride, and the protic solvent is selected from C₁₋₄ alcohols.

9. The method according to claim 7 or 8, wherein the compound of formula (4) is prepared by the following steps: 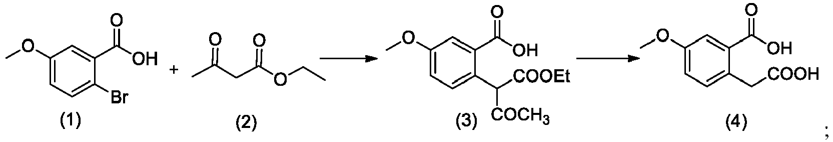 wherein,
a compound of formula (1) is reacted with a compound of formula (2) in the presence of a catalyst and a strong base to give a compound of formula (3); and
the compound of formula (3) is reacted in the presence of a strong base to give the compound of formula (4);
wherein, the catalyst is selected from metal salts, and preferably CuBr, CuCl, CuI, FeCl₂, FeSO₄ and FeBr₂; the strong base is selected from NaOH, KOH, LiOH, NaH, KH and LiH.

10. Use of the impurity B of the compound of formula VI according to claim 1 in the quality control of the compound of formula VI, wherein the impurity B of the compound of formula VI is used as a control substance.

11. Use of the impurity G of the compound of formula VI according to claim 3 in the quality control of the compound of formula VI, wherein the impurity G of the compound of formula VI is used as a control substance.

12. Use of the impurity A of the compound of formula VI according to claim 6 in the quality control of the compound of formula VI, wherein the impurity A of the compound of formula VI is used as a control substance.

13. A compound of formula VI, wherein the content of the impurity B according to claim 1 in the compound of formula VI is 0.01-1 wt.%.

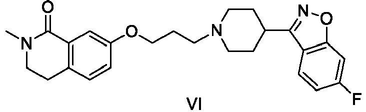

14. A compound of formula VI, wherein the content of the impurity G according to claim 3 in the compound of formula VI is 0.01-1 wt.%.

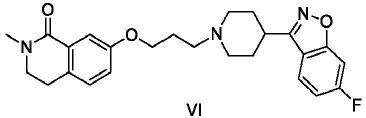

15. A compound of formula VI, wherein the content of the impurity A according to claim 6 in the compound of formula VI is 0.01-1 wt.%.
